# FASCICULE DE BREVET EUROPEEN

(11) **EP 4 541 396 B1**
(45) Date de publication et mention de la délivrance du brevet: **08.04.2026**
(21) Numéro de dépôt: 24206785.8
(22) Date de dépôt: 15.10.2024
(51) Int. Cl.: A61M 11/00, A61M 11/02, A61M 15/00, A61M 15/08

(54) **PULVÉRISATEUR NASAL ET PROCÉDÉ DE CHARGEMENT DU PULVÉRISATEUR NASAL**
NASENSPRÜHER UND VERFAHREN ZUM LADEN DES NASENSPRÜHERS
NASAL SPRAYER AND METHOD FOR LOADING THE NASAL SPRAYER

(30) Priorité: 16.10.2023 FR 2311138
(43) Date de publication de la demande: 23.04.2025
(73) Titulaire: Unither Pharmaceuticals, 75009 Paris (FR)
(72) Inventeur: MAURY, Marc, 33160 Saint-Médard-en-Jalles (FR)
(74) Mandataire: Regimbeau

(56) Documents cités:
- FR-A- 1 539 702
- FR-A- 1 585 647
- GB-A- 1 184 065
- US-A- 2 583 821
- US-A- 3 369 713
- US-A- 3 382 870
- US-A- 4 564 129

## Description

### DOMAINE DE L'INVENTION

La présente invention concerne un pulvérisateur nasal et un procédé de chargement d'un tel pulvérisateur.

### ETAT DE LA TECHNIQUE

Un pulvérisateur nasal conventionnel comprend un réservoir stockant un liquide à administrer et un embout nasal.

Certains pulvérisateurs s'utilisent de la façon suivante : l'utilisateur penche sa tête en arrière, place l'embout dans son nez, et expulse une partie du liquide contenue dans le pulvérisateur en pressant sur le réservoir. Toutefois, devoir pencher la tête en arrière est inconfortable. Par ailleurs, la quantité de liquide administrée dépend du niveau de pression exercé par l'utilisateur sur le réservoir. Ainsi, la quantité de liquide administrée n'est pas précise.

D'autres pulvérisateurs peuvent s'utiliser sans que l'utilisateur n'ait à pencher sa tête en arrière. Pour permettre l'expulsion d'une dose de liquide précise, ces pulvérisateurs comprennent des mécanismes complexes, utilisant typiquement un tube plongeur. Toutefois, la complexité de ces mécanismes rend la fabrication de ces pulvérisateurs onéreuse.

Des pulvérisateurs de l'art antérieur sont connus des documents US 3 382 870 A , GB 1 184 065 A, US 4 564 129 A, US 2 583 821 A et US 3 369 713 A.

### RÉSUMÉ DE L'INVENTION

La présente invention a pour objet un pulvérisateur nasal tel que défini dans la revendication indépendante 6, ainsi qu'un procédé de chargement d'un tel pulvérisateur tel que défini dans la revendication indépendante 1. Des modes de réalisation préférés de l'invention sont définis dans les revendications dépendantes.

### EXPOSE DE L'INVENTION

Un but de l'invention est de doser précisément une dose de liquide administrable à un utilisateur de manière confortable pour l'utilisateur, à l'aide d'un pulvérisateur peu onéreux.

Ce but est atteint par un procédé de chargement d'un pulvérisateur nasal, le pulvérisateur nasal comprenant : un liquide, un réservoir pour stocker le liquide, le réservoir comprenant deux parois propres à être déplacées l'une vers l'autre par déformation élastique, une sortie débouchant à l'extérieur du pulvérisateur nasal, et un doseur fluidiquement relié au réservoir et à la sortie de sorte que: le liquide s'écoule du réservoir vers le doseur par gravité, lorsque le pulvérisateur nasal est placé dans un première position dans laquelle le réservoir est au-dessus du doseur; le doseur retient une dose du liquide par capillarité et laisse une quantité excédentaire du liquide refluer vers le réservoir par gravité, lorsque le pulvérisateur nasal passe de la première position à une deuxième position dans laquelle le réservoir est en-dessous du doseur; le doseur expulse la dose de liquide vers la sortie de sorte que la dose de liquide quitte le pulvérisateur par la sortie, lorsque les deux parois sont déplacées l'une vers l'autre par déformation élastique pendant que le pulvérisateur nasal est dans la deuxième position; le pulvérisateur nasal étant caractérisé en ce que le liquide a un volume adapté pour que la quantité excédentaire de liquide ayant reflué dans le réservoir ne quitte pas le pulvérisateur nasal lorsque les deux parois sont déplacées l'une vers l'autre par déformation élastique jusqu'à se toucher pendant que le pulvérisateur nasal est dans la deuxième position, lors d'une première utilisation du pulvérisateur nasal par un utilisateur. Compresser les deux parois l'une vers l'autre cause une expulsion de la dose de liquide depuis le doseur vers la sortie puis hors du pulvérisateur nasal. Le procédé comprend les étapes suivantes :
- saisir le pulvérisateur nasal,
   - placer le pulvérisateur nasal dans une première position dans laquelle le réservoir est au-dessus du doseur, de sorte que liquide s'écoule du réservoir vers le doseur par gravité,
   - faire passer le pulvérisateur nasal de la première position à une deuxième position dans laquelle le réservoir est en dessous du doseur, de sorte que le doseur retienne une dose du liquide par capillarité et laisse une quantité excédentaire du liquide refluer par gravité vers le réservoir.

Le procédé peut comprendre les caractéristiques optionnelles suivantes, prises seules ou en combinaison à chaque fois que cela est techniquement possible.

De préférence, le procédé comprend une étape consistant à agiter le pulvérisateur nasal dans une direction transversale à un axe longitudinal le long duquel le réservoir et le doseur sont alignés, lorsque le pulvérisateur nasal est dans la première position.

De préférence, le doseur comprend un espace de stockage pour la dose de liquide, et dans lequel le liquide a un volume supérieur à deux fois le volume de l'espace stockage.

De préférence, le doseur comprend un espace de stockage pour la dose de liquide, l'espace de stockage ayant un volume compris entre 20 et 120 microlitres.

Il est également proposé un pulvérisateur nasal comprenant : un liquide, un réservoir pour stocker le liquide, le réservoir comprenant deux parois propres à être déplacées l'une vers l'autre par déformation élastique, une sortie débouchant à l'extérieur du pulvérisateur nasal et un doseur. Le doseur est fluidiquement relié au réservoir et à la sortie de sorte que :
- le liquide s'écoule du réservoir vers le doseur par gravité, lorsque le pulvérisateur nasal est placé dans une première position dans laquelle le réservoir est au-dessus du doseur,
- le doseur retient une dose du liquide par capillarité et laisse une quantité excédentaire du liquide refluer vers le réservoir par gravité, lorsque le pulvérisateur nasal passe de la première position à une deuxième position dans laquelle le réservoir est en-dessous du doseur,
- le doseur expulse la dose de liquide vers la sortie de sorte que la dose de liquide quitte le pulvérisateur par la sortie, lorsque les deux parois sont déplacées l'une vers l'autre par déformation élastique pendant que le pulvérisateur nasal est dans la deuxième position.

Le liquide a un volume adapté pour que la quantité excédentaire de liquide ayant reflué dans le réservoir ne quitte pas le pulvérisateur nasal lorsque les deux parois sont déplacées l'une vers l'autre par déformation élastique jusqu'à se toucher pendant que le pulvérisateur nasal est dans la deuxième position, lors d'une première utilisation du pulvérisateur nasal par un utilisateur.

Le pulvérisateur peut comprendre les caractéristiques optionnelles suivantes, prises seules ou en combinaison à chaque fois que cela est techniquement possible.

De préférence, le réservoir, le doseur et la sortie sont alignés le long d'un axe longitudinal. De préférence, le pulvérisateur nasal comprend un flacon, le flacon comprenant un fût formant le réservoir et un col formant le doseur.

De préférence, le pulvérisateur nasal comprend un embout dans lequel la sortie est formée, l'embout étant détachable du doseur et/ou du réservoir.

De préférence, le doseur comprend un espace de stockage pour la dose de liquide ayant un premier diamètre, le pulvérisateur comprenant par ailleurs un canal pour acheminer la dose de liquide depuis le doseur jusqu'à la sortie, le canal ayant un deuxième diamètre interne inférieur au premier diamètre.

### DESCRIPTION DES FIGURES

D'autres caractéristiques, buts et avantages de l'invention ressortiront de la description qui suit, qui est purement illustrative et non limitative, et qui doit être lue en regard des dessins annexés sur lesquels :
La figure 1 est une vue en coupe schématique d'un pulvérisateur nasal selon un mode de réalisation.
La figure 2 est une vue en coupe détaillant une partie du pulvérisateur de la figure 1.
La figure 3a, la figure 3b, la figure 3c et la figure 3d sont des vues en coupes du pulvérisateur de la figure 1 à différents stades d'un procédé de pulvérisation nasale.

Sur l'ensemble des figures, les éléments similaires portent des références identiques.

### DESCRIPTION DETAILLEE

### 1) Pulvérisateur nasal

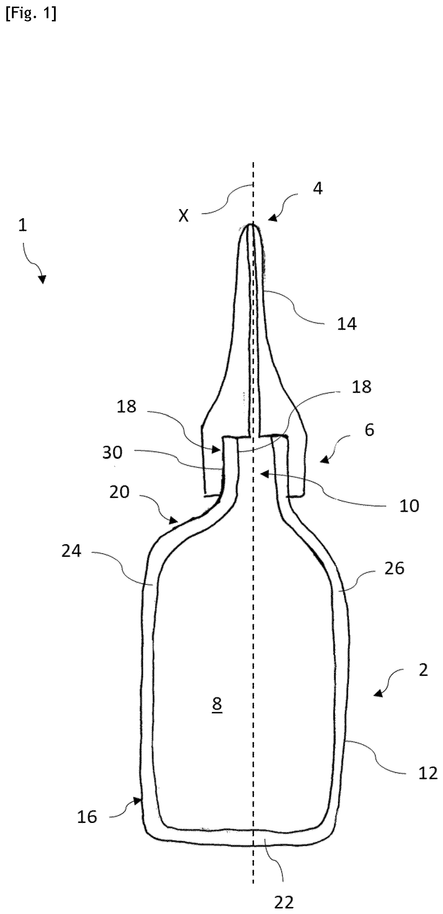

En référence à la figure 1, un pulvérisateur nasal 1 comprend un réservoir 2, une sortie 4 débouchant à l'extérieur du pulvérisateur nasal 1, et un doseur 6 fluidiquement relié au réservoir 2 et à la sortie 4.

Le réservoir 2 a pour fonction de stocker un liquide destiné à être pulvérisé dans le nez d'un utilisateur. Le réservoir 2 comprend un espace de stockage principal 8 pour ce liquide.

Le doseur 6 est fluidiquement relié au réservoir 2. Le doseur 6 comprend un espace de stockage secondaire 10. L'espace de stockage secondaire 10 a un volume inférieur à celui de l'espace de stockage principal 8. En conséquence, le doseur 6 a une capacité inférieure à la capacité du réservoir 2.

Dans la présente divulgation, on appelle « dose de liquide » une quantité de liquide susceptible d'être stockée dans le doseur 6.

La figure 1 montre le pulvérisateur positionné dans une position dite « vers le haut », dans laquelle le réservoir 2 est en-dessous du doseur 6.

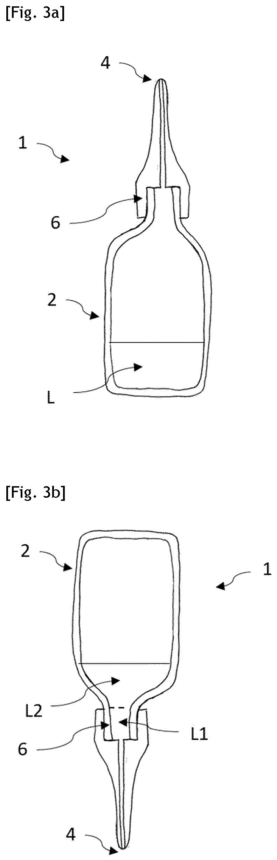

Le pulvérisateur peut également être position dans une position dite « vers le bas », dans laquelle le réservoir est au-dessus du doseur 6 (voir figure 3b).

Dans la position vers le bas, un liquide initialement contenu dans le réservoir 2 peut s'écouler du réservoir 2 vers le doseur 6 par simple gravité. De la sorte, une portion du liquide en provenance du réservoir 2 peut atteindre le doseur 6.

Si la quantité totale de liquide stockée dans le le pulvérisateur 1 a un volume supérieur à la capacité du doseur 6, alors la dose de liquide ne représente qu'une fraction de cette quantité totale. Ainsi, dans la première position, une portion excédentaire de cette quantité totale de liquide demeure à l'extérieur du doseur 6.

Si à l'opposé la quantité totale de liquide stocké dans le pulvérisateur 1 a un volume inférieur ou égal à la capacité du doseur 6, alors il est possible de transvaser tout le liquide dans le doseur 6, et la dose de liquide correspond alors à la quantité totale de liquide stocké dans le pulvérisateur 1. Dans ce cas, il n'y a pas de portion excédentaire de liquide à l'extérieur du doseur 6, lorsque le pulvérisateur 1 est dans la première position.

Le doseur 6 est configuré pour retenir une dose du liquide qu'il stocke par capillarité, lorsque le pulvérisateur 1 passe de la position vers le bas à la position vers le haut. Si la quantité totale de liquide stocké dans le pulvérisateur 1 a un volume supérieur à la capacité du doseur 6, alors le doseur 6 laisse une quantité excédentaire du liquide refluer vers le réservoir 2 par simple gravité.

Le doseur 6 est par ailleurs relié fluidiquement à la sortie 4 du pulvérisateur 1.

Le doseur 6 est agencé entre le réservoir 2 et la sortie 4. De la sorte, un liquide initialement stocké par le réservoir 2 passe par le doseur 6 avant d'atteindre la sortie 4, et ce liquide est expulsé le pulvérisateur nasal 1 par cette sortie 4 (on détaillera dans la suite comme cette expulsion est réalisée).

Le pulvérisateur nasal 1 présente une forme allongée le long d'un axe longitudinal X. Le réservoir 2, le doseur 6 et la sortie 4 sont alignés le long de cet axe longitudinal X. Ainsi, lorsque du liquide s'écoule depuis le réservoir vers le doseur ou depuis le doseur vers la sortie, la direction moyenne d'écoulement de ce liquide est parallèle à l'axe longitudinal X.

Le pulvérisateur nasal 1 comprend un flacon 12 et un embout nasal 14.

Le flacon 12 et l'embout 14 constituent deux parties du pulvérisateur 1 susceptible d'être mises en prise l'une avec l'autre, et d'être détachées l'une de l'autre.

Le flacon 12 a une forme allongée le long de l'axe longitudinal X.

Le flacon 12 comprend un fût 16 et un col 18. Le fût 16 et le col 18 sont reliée par un épaulement 20, de sorte que le col 18 présente un diamètre interne inférieur à celui du fût 16.

Le fût 16 forme le réservoir 2 discuté précédemment.

Le réservoir 2 comprend une base 22 et deux parois déformables 24, 26 délimitant entre elles l'espace de stockage principal 8.

La base 22 est une paroi s'étend transversalement à l'axe longitudinal X. La base 22 a une surface interne formant un fond de l'espace de stockage principal 8, et une surface externe opposée à la surface interne, cette surface externe constituant une extrémité inférieure du flacon 12 lorsque le flacon 12 placé dans la position vers le haut (représentée en figure 1).

Les deux parois déformables 24, 26 ont une position de repos dans laquelle les deux parois sont à distance l'une de l'autre. Par exemple, dans cette position de repos, les deux parois déformables 24, 26 s'étendent parallèlement à l'axe longitudinal X.

Les deux parois déformables 24, 26 peuvent être compressées l'une vers l'autre dans une direction transversale (perpendiculaire à l'axe longitudinal X), et ainsi se rapprocher l'une de l'autre par déformation élastique.

Sous l'effet d'une telle compression, les deux parois déformables 24, 26 sont aptes à passer de leur position de repos à une position déformée dans laquelle les deux parois déformables 24, 26 se touchent. Au cours de cette déformation élastique, le volume de l'espace de stockage principal se réduit, et ceci permet d'expulser un liquide contenu dans le réservoir 2 hors du flacon 12 (et même hors du pulvérisateur nasal 1), comme on le verra plus en détail dans la suite.

Le flacon 12 est d'une seule pièce.

Le flacon 12 est réalisé de préférence dans un élastomère.

Le flacon 12 peut être obtenu par un procédé de formage-remplissage-scellage (« blow-fill-seal » en anglais) ou par d'autres techniques de plasturgie, comme une injection, une injection-soufflage, ou une extrusion-soufflage.

Le col 18 délimite un orifice de sortie du flacon 12 (cet orifice est un orifice supérieur lorsque le pulvérisateur est dans la deuxième position montrée en figure 1).

Le col 18 présente une surface interne 28 s'étendant autour de l'axe longitudinal X. La surface interne 28 du col 18 délimite l'espace de stockage secondaire 10 pour stocker une dose de liquide.

La surface interne 28 du col 18 est par exemple tronconique ou cylindrique.

Le col 18 comprend par ailleurs une surface externe 30 opposée à la surface interne 28.

Le flacon 12 comprend en outre un filetage externe. Le filetage externe est formé dans la surface externe du col 18. Le filetage externe est propre à coopérer avec l'embout nasal 14.

Le doseur 6 discuté précédemment est formé par le col 18 du flacon 12. L'espace de stockage secondaire 10, destiné à recevoir une dose de liquide, est délimité par la surface interne du col 18.

De préférence, l'espace de stockage secondaire 10 a un volume compris entre 20 et 120 microlitres. Pour obtenir un tel volume tout en obtenant un effet de capillarité dans cet espace de stockage secondaire 10, de sortie 4 à y retenir une dose, l'homme du métier tiendra compte principalement des paramètres suivants : diamètre de l'espace délimité par la surface interne (ce diamètre étant mesuré perpendiculairement à l'axe longitudinal X), et longueur de cet espace (mesurée parallèlement à l'axe longitudinal X), viscosité du liquide.

L'embout nasal 14 comprend une surface interne 32 délimitant une cavité pour recevoir le col 18 du flacon 12. La surface interne 32 s'étend autour de l'axe longitudinal X.

L'embout nasal 14 comprend par ailleurs un filetage interne. Le filetage interne est formé dans la surface externe 32, et est adapté pour coopérer avec le filetage externe du flacon 12. Pour assembler le flacon 12 et l'embout nasal 14, un utilisateur visse l'embout nasal 14 autour du col 18, en mettant en prise le filetage interne et le filetage externe. Le vissage s'effectue par rotation de l'embout 14 par rapport au flacon 12, autour de l'axe longitudinal X.

L'embout 14 présente une extrémité distale destinée à être insérée dans le nez d'un utilisateur. La sortie 4 discutée précédemment est formée à cette extrémité distale.

L'embout nasal 14 comprend également un canal 34 reliant la cavité à la sortie 4. Le canal 34 est rectiligne. Il s'étend le long de l'axe longitudinal X.

Lorsque l'embout nasal 14 et le flacon 12 sont assemblés, le canal 34 est en communication fluidique avec le doseur 6 : ce canal 34 prolonge ainsi l'espace de stockage secondaire 10 délimité par le doseur 6. Plus précisément, le canal 34 est aligné avec l'orifice de sortie 4 du flacon 12.

Le canal 34 présente un diamètre interne inférieur à celui du doseur (c'est-à-dire au diamètre de l'espace de stockage secondaire 10 délimité par la surface interne 18.

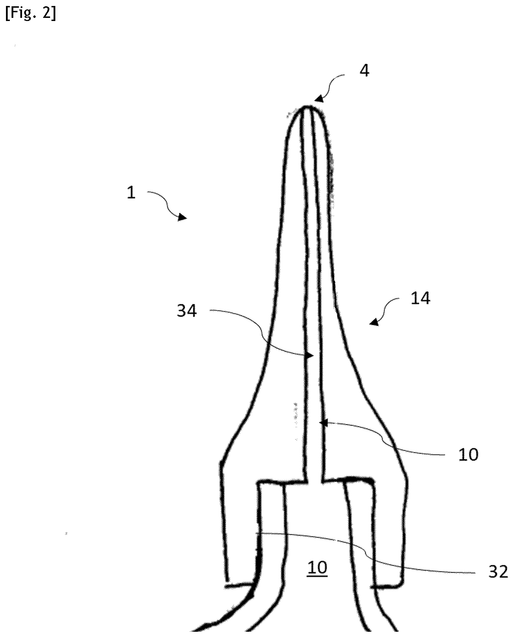

Le flacon 12 représenté sur les figures 1 et 2 est dans un état ouvert (du liquide peut sortir du flacon par le col 18). Toutefois, le col peut être scellé par une partie de scellage avant sa première utilisation (no représentée). La partie de scellage est adaptée pour pouvoir être rompue par un utilisateur, par exemple un mouvement de rotation, et être ainsi détachée du reste du flacon 12 de sorte à former une ouverture au niveau du col donnant accès à l'intérieur du flacon 12 depuis l'extérieur du flacon 12. Le flacon est alors dans un état ouvert. Une fois ouvert, le flacon 12 peut être mis en prise avec l'embout 14 comme indiqué précédemment, par coopération des filetages susmentionnés.

### 2) Procédé de chargement du pulvérisateur nasal

Un procédé de chargement du pulvérisateur 1 décrit précédemment comprend les étapes suivantes. Le chargement a pour but de préparer une dose de liquide au sein du pulvérisateur 1.

On suppose que le flacon 12 contient un liquide L dont le volume est supérieur à N doses de liquide, avec N≥1, de préférence N>1, voire N>2.

Un utilisateur saisit le pulvérisateur 1. On suppose dans un premier temps que le pulvérisateur est dans la position vers le haut (voir figure 3a). Le liquide L est alors situé au fond du réservoir 2, et le doseur 6 est vide.

L'utilisateur place le pulvérisateur dans la position vers le bas (voir figure 3b). Le réservoir 2 est alors est au-dessus du doseur 6. Le liquide L s'écoule alors du réservoir 2 vers le doseur 6 par gravité, si bien que le doseur 6 se remplit de liquide. Le liquide ne s'écoule depuis le doseur vers la sortie 4 par gravité. Une dose L1 du liquide L pénètre dans le doseur 6. Une quantité excédentaire L2 du liquide L demeure à l'extérieur du doseur 6, dans le réservoir 2. A ce stade, il n'y a pas de séparation physique entre la dose L1 et la quantité excédentaire L2 (ce qui est reflété par la ligne horizontale en pointillés sur la figure 3b).

Avantageusement, l'utilisateur agite le pulvérisateur nasal 1 dans une direction transversale à l'axe longitudinal X lorsque le pulvérisateur nasal 1 est dans la position vers le bas. Une telle agitation permet d'évacuer d'éventuelles bulles d'air hors du doseur 6, vers le réservoir 2, ce qui garantit que le doseur 6 reçoit précisément une dose L1 de liquide, et non une quantité de liquide inférieure à une dose en raison de la présence de bulles d'air dans l'espace de stockage secondaire 10. Ainsi, l'agitation contribue rendre plus précise la dose de liquide à administrer à l'utilisateur.

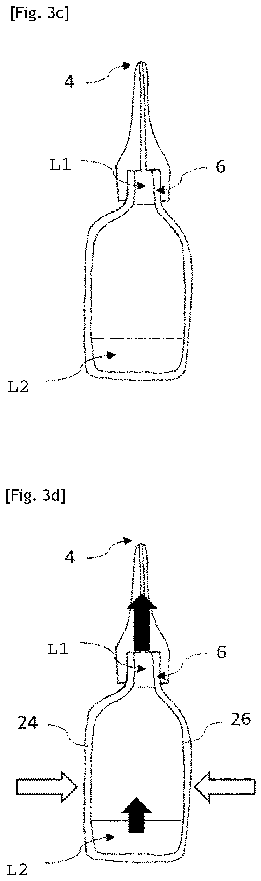

L'utilisateur fait ensuite passer le pulvérisateur 1 de la position vers le bas à la position vers le haut (voir en figure 3c). Le réservoir 2 passe en dessous du doseur 6. Le doseur 6 retient la dose L1 du liquide par capillarité et laisse la quantité excédentaire L2 du liquide refluer par gravité vers le fond du réservoir 2. Le liquide L contenu dans le pulvérisateur 1 est alors séparé en deux portions : d'une part, la dose de liquide L1 reste dans le doseur 6, et d'autre part la quantité excédentaire de liquide L2, qui repose au fond du réservoir 2, avec un espace entre les deux portions susmentionnées.

Le chargement du pulvérisateur 1 est alors terminé.

### 3) Procédé de pulvérisation nasale

Un procédé de pulvérisation nasale à l'aide du pulvérisateur 1 chargé comme indiqué ci-dessus comprend les étapes suivantes.

L'utilisateur place l'embout 14 dans son nez.

L'utilisateur presse ensuite sur les parois déformables 24, 26 du réservoir 2, de sorte à les rapprocher l'une de l'autre par déformation élastique dans une direction transversale perpendiculaire à l'axe longitudinal X (voir les deux flèches blanches représentée sur la figure 3d). Cette déformation élastique réduit le volume de l'espace de stockage principal 8, et cause une surpression dans le réservoir. Cette suppression cause une expulsion de la dose retenue dans le doseur 6 vers la sortie 4, en passant par le canal 34 (cette expulsion étant représentée sur la figure 3d par une flèche noire au niveau du canal 34).

L'utilisateur presse sur les parois déformables 24, 26 jusqu'à ce que la dose initialement retenue dans le dose quitte entièrement le pulvérisateur 1 par la sortie 4, et pénètre ainsi dans son nez. L'utilisateur peut exercer cette pression par exemple avec le pouce et l'index. L'utilisateur peut éventuellement sentir avec ses doigts le moment ou la dose finit d'être expulsée par la sortie 4. A défaut, l'utilisateur peut presser sur les parois déformables 24, 26 jusqu'à ce que les parois se touchent mutuellement.

Au cours de la déformation élastique, le niveau du liquide au fond du réservoir est susceptible de monter (voir la flèche noire inférieure sur la figure 3d), en direction de la sortie 4.

Lorsque l'utilisateur cesse de presser sur les parois déformables 24, 26, ces parois 24, 26 s'écartent l'une de l'autre par retour élastique, et reviennent dans leur position de repos, à distance l'une de l'autre. L'espace de stockage principal retrouve alors son volume initial.

S'il reste assez de liquide dans le pulvérisateur, l'utilisateur peut répéter les étapes qui précède pour s'administrer une nouvelle dose de liquide.

Le fait que l'utilisateur presse sur les deux parois déformables 24, 26 alors que le pulvérisateur 1 est dans la position vers le haut, dans laquelle le doseur 6 est au-dessus du réservoir 2, contribue à rentre l'administration précise d'une dose à l'utilisateur. En effet, c'est dans cette position qu'il y a une séparation physique entre la dose retenue dans le doseur 6 et la quantité excédentaire de liquide ayant refluée dans le réservoir 2, comme représenté en figure 3c. Réaliser la pression sur les deux parois déformables 24, 26 alors que le pulvérisateur 1 est dans la position vers le haut a pour avantage supplémentaire d'être confortable pour l'utilisateur, car permet une administration d'une dose de liquide dans le nez de l'utilisateur sans que l'utilisateur n'ait à pencher la tête en arrière.

Une situation qu'il est souhaitable d'éviter est d'expulser non seulement la dose contenue dans le doseur 6 mais également une partie de la quantité excédentaire de liquide L2 se trouvant dans le réservoir 2. Ceci pourrait survenir si l'utilisateur ne sentait pas au toucher que la dose de liquide L2 vient de complètement quitter le pulvérisateur 1, et qu'il continuait alors à presser les parois 24, 26 l'une vers l'autre, si bien que la montée du liquide L2 se poursuivrait jusqu'à atteindre la sortie 4. Dans une telle situation, une surdose risquerait administrée à l'utilisateur.

Pour éviter une telle situation, le volume de liquide contenu dans le pulvérisateur 1 est ajusté pour que la portion excédentaire de liquide L2 ayant reflué dans le réservoir 2 ne quitte pas le pulvérisateur 1 lorsque les deux parois sont déplacées l'une vers l'autre par déformation élastique dans la position déformée dans laquelle les deux parois se touchent.

Cette caractéristique garantit avantageusement d'éviter l'administration d'une surdose de liquide, car la position déformée constitue une position extrémale pour les parois, que l'utilisateur est capable de nettement sentir avec ses doigts.

Selon l'invention, il est fait en sorte que cette condition soit satisfaite à la première utilisation du pulvérisateur 1 par un utilisateur (c'est-à-dire lors de la première mise en œuvre des étapes exposées ci-dessus).

Dans un mode de réalisation, le volume de liquide est strictement inférieur à cinq doses. Ceci implique que le pulvérisateur ne peut être utilisé que pour administrer quatre doses ayant un volume correspondant à l'espace de stockage du doseur, par exemple deux doses par narine. Un excédent de liquide peut rester dans le réservoir après l'administration de la quatrième dose, mais cet excédent n'est pas suffisant pour remplir complètement le doseur et ainsi retenir une cinquième dose ayant le même volume que les quatre doses déjà administrées.

Dans un autre mode de réalisation, le volume de liquide dans le pulvérisateur est strictement inférieur à trois doses. Ceci implique que le pulvérisateur nasal peut être utilisé pour administrer deux doses ayant un volume correspondant à l'espace de stockage du doseur, par exemple une seule dose par narine. Un excédent de liquide peut rester dans le réservoir après l'administration de la deuxième dose, mais cet excédent n'est pas suffisant pour remplir le doseur et ainsi retenir une troisième dose ayant le même volume que les deux doses déjà administrées.

Le pulvérisateur 1 décrit précédemment peut faire l'objet de variantes.

Dans le mode de réalisation représenté sur les figures, le pulvérisateur ne comprend que deux pièces (le flacon 14 et l'embout 16). Dans d'autres modes de réalisation, le pulvérisateur 1 comprend un nombre de pièces différent. Par exemple, le réservoir 2, la sortie 4 et le doseur 6 peuvent être formés dans trois pièces différentes et pouvant être assemblées.

Par ailleurs d'autres moyens que des filetages peuvent être prévus pour mettre en prise le flacon 12 avec l'embout 14.

## Revendications

1. Procédé de chargement d'un pulvérisateur nasal (1) selon la revendication 6, le procédé comprenant les étapes suivantes :
- saisir un pulvérisateur nasal (1) selon la revendication 6,
• placer le pulvérisateur nasal (1) dans une première position dans laquelle le réservoir (2) est au-dessus du doseur (6), de sorte que liquide s'écoule du réservoir (2) vers le doseur (6) par gravité,
• faire passer le pulvérisateur nasal (1) de la première position à une deuxième position dans laquelle le réservoir (2) est en dessous du doseur (6), de sorte que le doseur (6) retienne une dose (L1) du liquide par capillarité et laisse une quantité excédentaire (L2) du liquide refluer par gravité vers le réservoir (2).

2. Procédé selon la revendication précédente, dans lequel le volume du liquide est strictement inférieur à cinq doses, voire strictement inférieur à trois doses.

3. Procédé selon l'une quelconque des revendications précédentes, comprenant agiter le pulvérisateur nasal (1) dans une direction transversale à un axe longitudinal (X) le long duquel le réservoir (2) et le doseur (6) sont alignés, lorsque le pulvérisateur nasal (1) est dans la première position.

4. Procédé selon l'une quelconque des revendications précédentes, dans lequel le doseur (6) comprend un espace de stockage (10) pour la dose de liquide, et dans lequel le liquide (L) a un volume supérieur à deux fois le volume de l'espace stockage (10).

5. Procédé selon l'une quelconque des revendications précédentes, dans lequel le doseur (6) comprend un espace de stockage (10) pour la dose de liquide, l'espace de stockage (10) ayant un volume compris entre 20 et 120 microlitres.

6. Pulvérisateur nasal (1) comprenant :
• un liquide (L),
• un réservoir (2) pour stocker le liquide, le réservoir (2) comprenant deux parois propres à être déplacées l'une vers l'autre par déformation élastique,
• une sortie (4) débouchant à l'extérieur du pulvérisateur nasal (1),
• un doseur (6) fluidiquement relié au réservoir (2) et à la sortie (4) de sorte que :
• le liquide s'écoule du réservoir (2) vers le doseur par gravité, lorsque le pulvérisateur nasal (1) est placé dans une première position dans laquelle le réservoir (2) est au-dessus du doseur,
• le doseur retient une dose (L1) du liquide par capillarité et laisse une quantité excédentaire (L2) du liquide refluer vers le réservoir (2) par gravité, lorsque le pulvérisateur nasal (1) passe de la première position à une deuxième position dans laquelle le réservoir (2) est en-dessous du doseur,
• le doseur expulse la dose de liquide vers la sortie (4) de sorte que la dose de liquide quitte le pulvérisateur par la sortie (4), lorsque les deux parois sont déplacées l'une vers l'autre par déformation élastique pendant que le pulvérisateur nasal (1) est dans la deuxième position,
le pulvérisateur nasal étant **caractérisé en ce que** :
• le liquide (L) a un volume adapté pour que la quantité excédentaire (L2) de liquide ayant reflué dans le réservoir (2) ne quitte pas le pulvérisateur nasal (1) lorsque les deux parois sont déplacées l'une vers l'autre par déformation élastique jusqu'à se toucher pendant que le pulvérisateur nasal (1) est dans la deuxième position, lors d'une première utilisation du pulvérisateur nasal par un utilisateur.

7. Pulvérisateur nasal (1) selon la revendication précédente, dans lequel le volume du liquide est strictement inférieur à cinq doses, voire strictement inférieur à trois doses.

8. Pulvérisateur nasal (1) selon l'une quelconque des revendications 6 et 7, dans lequel le réservoir (2), le doseur et la sortie (4) sont alignés le long d'un axe longitudinal (X).

9. Pulvérisateur nasal (1) selon l'une quelconque des revendications 6 à 8, comprenant un flacon (12), le flacon (12) comprenant un fût (16) formant le réservoir (2) et un col (18) formant le doseur (6).

10. Pulvérisateur selon la revendication précédente, dans lequel le flacon (12) est obtenu par un procédé de formage-remplissage-scellage.

11. Pulvérisateur nasal (1) l'une quelconque des revendications 6 à 10, comprenant un embout (14) dans lequel la sortie (4) est formée, l'embout (14) étant détachable du doseur (6) et/ou du réservoir (2).

12. Pulvérisateur nasal (1) selon la revendication précédente, dans lequel le doseur (6) comprend un espace de stockage (10) pour la dose de liquide ayant un premier diamètre, le pulvérisateur comprenant par ailleurs un canal (34) pour acheminer la dose de liquide depuis le doseur jusqu'à la sortie (4), le canal (34) ayant un deuxième diamètre interne inférieur au premier diamètre.

## Patentansprüche

1. Verfahren zum Laden eines Nasensprühers (1) nach Anspruch 6,
wobei das Verfahren die folgenden Schritte umfasst:
• Ergreifen eines Nasensprühers (1) nach Anspruch 6,
• Platzieren des Nasensprühers (1) in eine erste Position, in der sich der Behälter (2) oberhalb des Dosierers (6) befindet, so dass die Flüssigkeit vom Behälter (2) zum Dosierer (6) durch Schwerkraft fließt,
• Wechseln des Nasensprühers (1) aus der ersten Position in eine zweite Position, in der sich der Behälter (2) unterhalb des Dosierers (6) befindet, so dass der Dosierer (6) eine Dosis (L1) der Flüssigkeit durch Kapillarität zurückhält und eine überschüssige Menge (L2) der Flüssigkeit durch Schwerkraft zum Behälter (2) zurückströmen lässt.

2. Verfahren nach vorstehendem Anspruch, wobei das Volumen der Flüssigkeit strikt kleiner als fünf Dosen, ja sogar strikt kleiner als drei Dosen ist.

3. Verfahren nach einem der vorstehenden Ansprüche, umfassend das Schütteln des Nasensprühers (1) in einer Richtung quer zu einer Längsachse (X), entlang der der Behälter (2) und der Dosierer (6) ausgerichtet sind, wenn der Nasensprüher (1) in der ersten Position ist.

4. Verfahren nach einem der vorstehenden Ansprüche, wobei der Dosierer (6) einen Vorratsbereich (10) für die Flüssigkeitsdosis umfasst, und wobei die Flüssigkeit (L) ein Volumen hat, das zweimal größer als das Volumen des Vorratsbereichs (10) ist.

5. Verfahren nach einem der vorstehenden Ansprüche, wobei der Dosierer (6) einen Vorratsbereich (10) für die Flüssigkeitsdosis umfasst, wobei der Vorratsbereich (10) ein Volumen hat, das zwischen 20 und 120 Mikrolitern liegt.

6. Nasensprüher (1), umfassend:
• eine Flüssigkeit (L),
• einen Behälter (2) zum Bevorraten der Flüssigkeit, wobei der Behälter (2) zwei Wände umfasst, die durch elastische Verformung zueinander bewegbar sind,
• einen Auslass (4), der außerhalb des Nasensprühers (1) ausmündet,
• eine Dosierer (6), der mit dem Behälter (2) und mit dem Auslass (4) fluidisch verbunden ist, so dass:
• die Flüssigkeit vom Behälter (2) zum Dosierer durch Schwerkraft fließt, wenn der Nasensprüher (1) in einer ersten Position platziert ist, in der der Behälter (2) oberhalb des Dosierers ist,
• der Dosierer eine Dosis (L1) der Flüssigkeit durch Kapillarität zurückhält und eine überschüssige Menge (L2) der Flüssigkeit durch Schwerkraft zum Behälter (2) zurückströmen lässt, wenn der Nasensprüher (1) aus der ersten Position in eine zweite Position wechselt, in der der Behälter (2) unterhalb des Dosierers ist,
• der Dosierer die Flüssigkeitsdosis zum Auslass (4) ausstößt, so dass die Flüssigkeitsdosis den Sprüher durch den Auslass (4) verlässt, wenn die beiden Wände durch elastische Verformung zueinander bewegt werden, während der Nasensprüher (1) in der zweiten Position ist,
wobei der Nasensprüher **dadurch gekennzeichnet** ists, dass:
• die Flüssigkeit (L) ein Volumen hat, das geeignet ist, damit die überschüssige Menge (L2) Flüssigkeit, die in den Behälter (2) zurückgeströmt ist, den Nasensprüher (1) nicht verlässt, wenn die beiden Wände durch elastische Verformung zueinander bewegt werden, bis sie sich berühren, während der Nasensprüher (1) in der zweiten Position ist, bei einer ersten Benutzung des Nasensprühers durch einen Benutzer.

7. Nasensprüher (1) nach vorstehendem Anspruch, wobei das Volumen der Flüssigkeit strikt kleiner als fünf Dosen, ja sogar strikt kleiner als drei Dosen ist.

8. Nasensprüher (1) nach einem der Ansprüche 6 und 7, wobei der Behälter (2), der Dosierer und der Auslass (4) entlang einer Längsachse (X) ausgerichtet sind.

9. Nasensprüher (1) nach einem der Ansprüche 6 bis 8, umfassend eine Flasche (12), wobei die Flasche (12) einen Schaft (16) umfasst, der den Behälter (2) bildet, und einen Hals (18), der den Dosierer (6) bildet.

10. Sprüher nach vorstehendem Anspruch, wobei die Flasche (12) durch ein Form-Fill-Seal-Verfahren erhalten wird.

11. Nasensprüher (1) nach einem der Ansprüche 6 bis 10, umfassend eine Spitze (14), in der der Auslass (4) gebildet ist, wobei die Spitze (14) vom Dosierer (6) und/oder vom Behälter (2) abnehmbar ist.

12. Nasensprüher (1) nach vorstehendem Anspruch, wobei der Dosierer (6) einen Vorratsbereich (10) für die Flüssigkeitsdosis umfasst, der einen ersten Durchmesser hat, wobei der Sprüher im Übrigen einen Kanal (34) umfasst, um die Flüssigkeitsdosis vom Dosierer bis zum Auslass (4) zu befördern, wobei der Kanal (34) einen zweiten Innendurchmesser hat, der kleiner ist als der erste Durchmesser.

## Claims

1. Method for loading a nasal spray (1) according to claim 6, the method comprising the following steps:
• grasping a nasal spray (1) according to claim 6,
• placing the nasal spray (1) in a first position in which the reservoir (2) is above the dispenser (6), so that liquid flows from the reservoir (2) to the dispenser (6) by gravity,
• moving the nasal spray (1) from the first position to a second position in which the reservoir (2) is below the dispenser (6), so that the dispenser (6) retains a dose (L1) of the liquid by capillary action and allows an excess quantity (L2) of the liquid to flow back by gravity to the reservoir (2),

2. Method according to the previous claim, in which the volume of liquid is strictly less than five doses, or even strictly less than three doses.

3. Method according to any of the preceding claims, comprising shaking the nasal spray (1) in a direction transverse to a longitudinal axis (X) along which the reservoir (2) and the dispenser (6) are aligned, when the nasal spray (1) is in the first position.

4. Method according to any of the preceding claims, wherein the dispenser (6) comprises a storage space (10) for the dose of liquid, and wherein the liquid (L) has a volume greater than twice the volume of the storage space (10).

5. Method according to any of the preceding claims, wherein the dispenser (6) comprises a storage space (10) for the dose of liquid, the storage space (10) having a volume between 20 and 120 microlitres.

6. Nasal spray (1) comprising:
• a liquid (L),
• a reservoir (2) for storing the liquid, the reservoir (2) comprising two walls capable of being moved towards each other by elastic deformation,
• an outlet (4) opening to the outside of the nasal spray (1),
• a dispenser (6) fluidically connected to the reservoir (2) and to the outlet (4) such that:
• the liquid flows from the reservoir (2) to the dispenser by gravity when the nasal spray (1) is placed in a first position in which the reservoir (2) is above the dispenser,
• the dispenser retains a dose (L1) of the liquid by capillary action and allows an excess quantity (L2) of the liquid to flow back to the reservoir (2) by gravity, when the nasal spray (1) moves from the first position to a second position in which the reservoir (2) is below the dispenser,
• the dispenser expels the dose of liquid towards the outlet (4) so that the dose of liquid leaves the sprayer through the outlet (4), when the two walls are moved towards each other by elastic deformation while the nasal sprayer (1) is in the second position,
the nasal sprayer being **characterised in that**:
• the liquid (L) has a volume adapted so that the excess quantity (L2) of liquid that has flowed back into the reservoir (2) does not leave the nasal spray (1) when the two walls are moved towards each other by elastic deformation until they touch while the nasal spray (1) is in the second position, during a first use of the nasal spray by a user.

7. Nasal spray (1) according to the previous claim, wherein the volume of liquid is strictly less than five doses, or even strictly less than three doses.

8. Nasal spray (1) according to any of claims 6 and 7, wherein the reservoir (2), the metering device and the outlet (4) are aligned along a longitudinal axis (X).

9. Nasal spray (1) according to any of claims 6 to 8, comprising a bottle (12), the bottle (12) comprising a barrel (16) forming the reservoir (2) and a neck (18) forming the dispenser (6).

10. Spray according to the previous claim, wherein the bottle (12) is obtained by a form-fill-seal process.

11. Nasal spray (1) according to any of claims 6 to 10, comprising a nozzle (14) in which the outlet (4) is formed, the nozzle (14) being detachable from the dispenser (6) and/or the reservoir (2).

12. Nasal spray (1) according to the previous claim, wherein the dispenser (6) comprises a storage space (10) for the dose of liquid having a first diameter, the spray lso comprising a channel (34) for conveying the dose of liquid from the dispenser to the outlet (4), the channel (34) having a second internal diameter smaller than the first diameter.
